# EUROPEAN PATENT SPECIFICATION

(11) **EP 3 498 276 B1**
(45) Date of publication and mention of the grant of the patent: **23.09.2020**
(21) Application number: 17382851.8
(22) Date of filing: 14.12.2017
(51) Int. Cl.: A61K 31/4164, A61K 31/505, A61P 15/02

(54) **ECTOINE AND ECTOINE DERIVATIVES FOR USE IN VULVOVAGINAL CONDITIONS**
ECTOIN UND ECTOIN-DERIVATE ZUR VERWENDUNG BEI VULVOVAGINALEN ZUSTÄNDEN
ECTOÏNE ET DE DÉRIVÉS D'ECTOÏNE À UTILISER DANS DES MALADIES VULVO-VAGINALES

(43) Date of publication of application: 19.06.2019
(73) Proprietor: Disproquima, S.A., 08227 Terrassa - Barcelona (ES)
(72) Inventor: MOLERO RODRÍGUEZ, Francisca, E-08921 Santa Coloma de Gramenet - Barcelona (ES); TOMASI SCIANÒ, Giuseppe Giovanni, E-20730 Azpeitia - Gipuzkoa (ES); JIMÉNEZ LÓPEZ, Oscar, E-08227 Terrassa - Barcelona (ES)
(74) Representative: ABG Intellectual Property Law, S.L.

(56) References cited:
- EP-A1- 0 664 124
- EP-A1- 0 671 161
- DE-A1- 19 911 775
- US-B1- 6 602 514

## Description

### Field of the Invention

The present invention relates to the use of ectoine and ectoine derivatives for the treatment and/or prevention of certain vulvovaginal dermatologic conditions.

### Background of the Invention

Vaginal problems are some of the most common reasons women go to the doctor. These problems can also affect the vulva, which is the external part of a woman's genitals.

Specifically, vulvovaginal atrophy, lichen sclerosus (lichen sclerosus et atrophicus), and Zoon's vulvitis are vulvovaginal dermatologic conditions which may be caused, inter alia, by an estrogen deficiency. Symptoms include vulvar itching, irritation, burning, dryness, and pain, which may be chronic or recurrent and can lead to significant physical discomfort and emotional distress and can affect mood and sexual relationships. With symptoms similar to common vaginal infections, women often seek care from gynecological providers and may be treated for vaginal infections without relief. Recognition and treatment of these vulvar conditions is important for symptom relief, sexual function, prevention of progressive vulvar scarring, and to provide surveillance for associated vulvar cancer.

Estrogen is essential for normal female sexual development and for the healthy functioning of the reproductive system.

Estrogen receptors are most abundant around genital areas, face, and lower limbs. So skin conditions involving these areas are more commonly affected in peri- and postmenopausal women.

A common intermediate complication of menopause is organ atrophy. **Vulvovaginal atrophy** is due to a vulvovaginal thinning. It often presents with vaginal dryness and burning, tenderness, pruritus, and dyspareunia. Urogenital atrophy occurs from rapid loss of collagen as a result of estrogen deficiency presenting with urgency, frequency, dysuria, painful urination, and incontinence. Declining collagen in the cardinal and uterosacral ligaments, caused by low estrogen levels, may cause uterine prolapse. **Lichen sclerosus (LS),** also known as lichen sclerosus et atrophicus (LSA), is a chronic atrophic skin disease that affects mainly anogenital areas in 85-93% cases involving both males and females. Prevalence rates are difficult to establish as it is underreported due to embarrassment as well as physician fail to recognize the disorder. Females are more commonly affected with 10:1 and 6:1 ratio in different series.

Most common symptoms of LS include intractable pruritus at night, irritation, painful intercourse, dysuria, urethral and vaginal discharge, dyspareunia, urinary and fecal incontinence, painful skin fissuring, and pruritus vulvae.

It presents with white atrophic polygonal, flat topped papules coalescing into plaques and extending into vulva and perianal skin in figure of eight configuration. Thickening of skin of vulva, perineum, labia majora, labia minora, fourchette, and clitoris may be seen. Sometimes clitoris disappears, labia shrink, and entrance to vagina tightens. It may present like cigarette paper with wrinkled surface and waxy thickened or fragile skin, bruises, blisters, purpura, telengectasia, erosion, or ulcers. It never affects inside vagina and cervix as it spares mucosa.

Skin around anus may be involved which aggravate the tendency of constipation as there is increased chances of genito-urinary infections.

Resolution of lesions is unlikely and is associated with increased risk of vulval cancer in 5% patients in the form of premalignant changes or squamous cell carcinom (SCC). LS can affect sites like inner thigh, buttocks, under breast, neck shoulders, and armpits.

**Zoon's vulvitis** is a rare, chronic condition of the vulva that presents with burning, pruritus, and dysuria with characteristic lesions and histopathology. Several treatment options have been reported with limited success.

All of the preceding conditions are extensively discussed in the prior art, although it is generally acknowledged that none has a specific known cause or remedy, and because their macroscopic and microscopic appearances are so variable and interchangeable, their precise diagnosis by clinical or histological techniques is extremely difficult. It has been found, however, that an estrogen deficiency like the natural decline of estrogen levels during menopause may represent a common cause.

Treatment of these conditions has become largely confined to the control of symptoms, primarily pruritus. In this respect, local application of corticosteroids (e.g. clobetasol) has been found to alleviate pruritus and swelling with some degree of success.

However, control of pruritus and burning does not assure that the lesions will regress.

Also current over-the-counter treatments include vaginal moisturizers for alleviating symptoms like pruritus and lubricants for dyspareunia. Vaginal moisturizers, which are water based, are available as liquids, gels, or ovules inserted every few days. Vaginal lubricants are shorter acting than moisturizers and are applied at the time of sexual activity to reduce dyspareunia.

Hormonal therapy, typically with vaginally administered local estrogens, is the standard of care for those patients that do not respond to other measures.

Finally, surgical treatment ranging from wide excision of a localized lesion to vulvectomy is utilized for those patients with uncontrollable disease. However, the high recurrence rate of the lesions associated with vulvar dystrophy requires regular observation of the patient for an extended period after surgery.

Accordingly, there is still a great need of providing therapeutic agents suitable for the treatment and/or prevention of vulvovaginal dermatologic conditions.

### Summary of the Invention

The present invention proposes the use of ectoine and ectoine derivatives for the treatment and/or prevention of vulvovaginal dermatologic conditions. As used herein, this term refers to conditions selected from the group consisting of vulvovaginal atrophy, lichen sclerosus (lichen sclerosus et atrophicus) or Zoon's vulvitis.

The inventors have demonstrated *in vivo* that the compound ectoine is able to successfully reduce labia fissures and other symptoms such as vaginal dryness, pruritus and dyspareunia that generally affect patients suffering from these vulvovaginal dermatologic conditions.

Therefore, in one aspect the present invention relates to a compound of the tautomeric formulae (I) or (II) 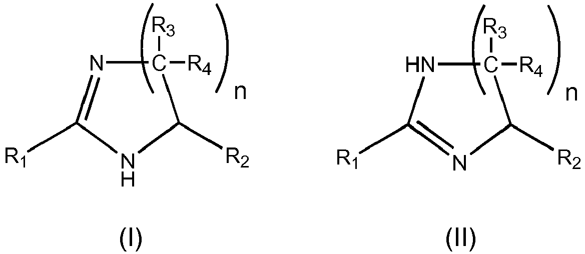
where R¹ is H or C₁-C₄ alkyl,
R² is H, -COOH, -COO(C₁-C₄ alkyl) or -CONHR⁵, where R⁵ is H, C₁-C₄ alkyl, an amino acid radical, dipeptide radical or tripeptide radical,
R³ and R⁴ are in each case independently of one another H or OH,
n is 1, 2 or 3,
or a pharmaceutically acceptable salt, stereoisomer or solvate thereof, for use in the treatment and/or prevention of a vulvovaginal dermatologic condition.

A further aspect is a pharmaceutical composition for use in the treatment and/or prevention of a vulvovaginal dermatologic condition, comprising a compound of formulae (I) or (II) or a pharmaceutically acceptable salt, stereoisomer or solvate thereof as defined above, and a pharmaceutically acceptable excipient. According to a particular embodiment, said composition is for topical administration.

Also is disclosed a method for the treatment and/or prevention of a vulvovaginal dermatologic condition in a subject comprising the administration of a therapeutically effective amount of a compound of formulae (I) or (II) or a pharmaceutically acceptable salt, stereoisomer or solvate thereof as defined above to said subject. In a preferred embodiment, said subject is a woman.

### Brief Description of the Drawings

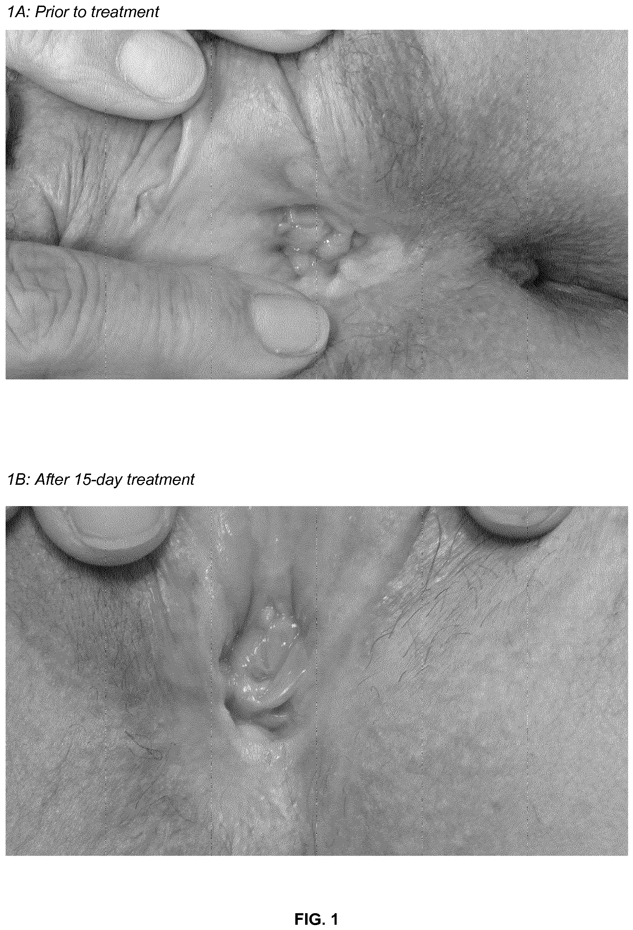

**Figure 1** shows photos of a patient's genitals before treatment (1A, top) and after a 15-day treatment with ectoine (1B, bottom).

### Detailed Description of the Invention

It is the object of the invention to provide an agent for the treatment and/or prevention of vulvovaginal dermatologic conditions. This object is accomplished as proposed in the present invention by the use of a compound according to formula (I) or (II) or a pharmaceutically acceptable salt, stereoisomer or solvate thereof.

Within the scope of the present invention, all compounds above and below chosen from the compounds of the formulae (I) and (II), the pharmaceutically acceptable salts, solvates and stereoisomeric forms thereof are referred to as "ectoine or ectoine derivatives".

The preparation of the compounds of the formulae (I) and (II) is described for instance in EP 0671161 A1.

By "pharmaceutically acceptable" is meant herein a material that is not biologically or otherwise undesirable, i. e., the material may be incorporated into a pharmaceutical composition administered to a patient without causing any undesirable biological effects or interacting in a deleterious manner with any of the other components of the composition in which it is contained. Preferably, as used herein, the term "pharmaceutically acceptable" means approved by a regulatory agency of the Federal or a state government or listed in the U.S. Pharmacopeia or other generally recognized pharmacopeia for use in animals, and more particularly in humans.

The term "salt" must be understood as any form of a compound used according to this invention in which said compound is in ionic form or is charged and coupled to a counterion (a cation or anion).

Examples of salts of particular acids are salts of: hydrochloric acid, hydrobromic acid, sulfuric acid, hydrobromide, monohydrobromide, monohydrochloride or hydrochloride, methiodide, methanesulfonic acid, formic acid, acetic acid, oxalic acid, succinic acid, malic acid, tartaric acid, mandelic acid, fumaric acid, lactic acid, citric acid, glutamic acid, hippuric acid, picric acid and/or aspartic acid. Examples of salts of particular bases are salts of alkali metals and alkaline earth metals and with NH₄.

The term "solvate" according to this invention is to be understood as meaning any form of the compounds according to the invention in which the compound has attached to it via non-covalent binding another molecule (most likely a polar solvent) especially including hydrates and alcoholates, e.g. methanolate. Methods of solvation are generally known within the art.

The term "stereoisomer" as used herein includes any enantiomer or diastereomer or such compound. In particular, compounds referred to herein may have asymmetric centres and therefore exist in different enantiomeric or diastereomeric forms. Thus any given compound referred to herein is intended to represent any one of a racemate, one or more enantiomeric forms, one or more diastereomeric forms, and mixtures thereof.

The alkyl groups include the methyl group CH₃, the ethyl group C₂H₅, the propyl groups CH₂CH₂CH₃ and CH(CH₃)₂ and the butyl groups CH₂CH₂CH₂CH₃, H₃CCHCH₂CH₃, CH₂CH(CH₃)₂ and C(CH₃)₃. One preferred alkyl group is the methyl group.

The term "amino acid" means the stereoisomeric forms, e.g. D and L forms, the following compounds: alanine, [beta]-alanine, arginine, asparagine, aspartic acid, cysteine, glutamine, glutamic acid, glycine, histidine, isoleucine, leucine, lysine, methionine, phenylaianine, serine, threonine, tryptophan, tyrosine, valine, [gamma]-aminobutyrate, N[epsilon]-acetyllysine, N[delta]-acetylornithine, N[gamma]-acetyldiaminobutyrate and N[alpha]-acetyldiaminobutyrate. L-amino acids are preferred.

Amino acid radicals are derived from the corresponding amino acids.

The radicals of the following amino acids are preferred: alanine, [beta]-alanine, asparagine, aspartic acid, glutamine, glutamic acid, glycine, serine, threonine, valine, [gamma]-aminobutyrate, N[epsilon]-acetyllysine, N[delta]-acetylornithine, N[gamma]-acetyldiaminobutyrate and N[alpha]-acetyldiaminobutyrate.

The di- and tripeptide radicals are acid amides according to their chemical nature and decompose upon hydrolysis into 2 or 3 amino acids. The amino acids in the di- and tripeptide radicals are bonded together by amide bonds. Preferred di- and tripeptide radicals are built up from the preferred amino acids.

In a particular embodiment, R¹ is H or methyl in formulae (I) or (II). Preferably, R¹ is methyl.

In another particular embodiment, R² is H or -COOH in formulae (I) or (II). Preferably, R² is -COOH.

In another particular embodiment, n is 2 in formulae (I) or (II).

In a preferred embodiment, the compound of formula (I) or (II) is selected from ectoine and hydroxyectoine or a pharmaceutically acceptable salt, stereoisomer or solvate thereof.

Ectoine and hydroxyectoine are tetrahydropyrimidine derivatives which are synthesized under stress conditions in extremophilic, especially halophilic microorganisms. Ectoine was detected in and subsequently isolated in 1985 from extremely halophilic species of the bacterial genus Ectothiorhodospira (Galinski et al. 1985). Hydroxyectoine (5-hydroxyectoine) may be synthesized from ectoine through a region- and stereo-specific hydroxylation reaction mediated by the EctD enzyme, a member of the non-heme-containing iron(II) and 2-oxoglutarate-dependent dioxygenases.

Ectoine and its derivatives are cytoprotectants widely synthesized by microorganisms as a defense against the detrimental effects of high osmolarity on cellular physiology and growth. These compounds possess the ability to preserve the functionality of proteins, macromolecular complexes, and even entire cells, attributes that led to their description as chemical chaperones. As a consequence, there is growing interest in using ectoines for biotechnological purposes, in skin care, and in medical applications.

Specifically, ectoine has a wide range of applications and is used for example as an active ingredient in skin care and sun protection products due to its ability to stabilize proteins and other cellular structures and protect the skin from stresses like UV irradiation and dryness.

The systematic name of ectoine is 2-methyl-1,4,5,6-tetrahydropyrimidine-4-carboxylic acid and of hydroxyectoine 5-hydroxy-2-methyl-1,4,5,6-tetrahydropyrimidine-4-carboxylic acid.

The structure of natural L-ectoine ((S)-2-methyl-1,4,5,6-tetrahydropyrimidine-4-carboxylic acid) is illustrated below:

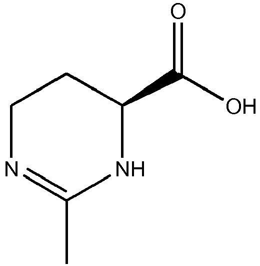

The structure of natural hydroxyectoine ((4S,5S)-5-hydroxy-2-methyl-1,4,5,6-tetrahydropyrimidine-4-carboxylic acid) is indicated hereunder:

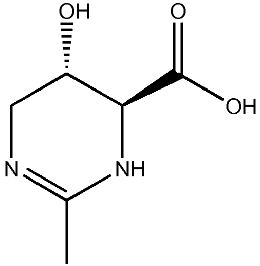

The use of the stereoisomers indicated is preferred but not obligatory, i.e. other stereoisomers or racemates may also be employed. Further, all the zwitterionic and tautomeric forms of ectoine and hydroxyectoine as well as any ectoine derivative are within the scope of the present invention.

Described herein is a new and surprising utility of ectoine and ectoine derivatives. Specifically, in this invention it is demonstrated that ectoine is able to successfully heal and reduce lesions and symptoms associated with a vulvovaginal dermatologic condition. Advantageously, topical application of ectoine in a patient suffering from lichen sclerosus quickly cured her painful labial fissuring and helped relieve other undesired vaginal symptoms including dryness and pruritus.

There has been to the inventors' knowledge no disclosure of the utility of ectoine in the treatment of any vulvovaginal dermatologic condition.

The present invention also relates to the use of a compound of formulae (I) or (II) or a pharmaceutically acceptable salt, stereoisomer or solvate thereof for the manufacture of a medicament for the treatment and/or prevention of a vulvovaginal dermatologic condition.

According to an embodiment of the present invention, ectoine or a derivative thereof is intended to heal or reduce the lesions and symptoms associated with a vulvovaginal dermatologic condition.

According to another embodiment of the present invention, ectoine or a derivative thereof is intended to heal or reduce labia fissures associated with a vulvovaginal dermatologic condition.

According to another embodiment of the present invention, ectoine or a derivative thereof is intended to heal or reduce vaginal dryness associated with a vulvovaginal dermatologic condition.

According to another embodiment of the present invention, ectoine or a derivative thereof is intended to heal or reduce vaginal pruritus associated with a vulvovaginal dermatologic condition.

According to another embodiment of the present invention, ectoine or a derivative thereof is intended to heal or reduce dyspareunia associated with a vulvovaginal dermatologic condition.

A further aspect is a pharmaceutical composition for use in the treatment and/or prevention of a vulvovaginal dermatologic condition comprising a compound of formulae (I) or (II) or a pharmaceutically acceptable salt, stereoisomer or solvate thereof, and a pharmaceutically acceptable excipient.

The compositions according to the invention are primarily intended for topical application and may be in any galenic form such as solutions, suspensions, emulsions, pastes, ointments, gels, creams, lotions, powders, soaps, surfactant-containing cleansing preparations, oils, foams and sprays.

Compositions in accordance with the invention include cosmetics, personal care products, cosmoceuticals and pharmaceutical preparations (medicaments).

The respective composition may - depending on its route of administration - also contain one or more excipients known to those skilled in the art. The composition or medicament according to the present invention may be produced according to standard procedures known to those skilled in the art.

The term "excipient" refers to components of a drug compound other than the active ingredient (definition obtained from the European Medicines Agency- EMA). They preferably include a "carrier, adjuvant and/or vehicle". Carriers are forms to which substances are incorporated to improve the delivery and the effectiveness of drugs. Drug carriers are used in drug-delivery systems such as the controlled-release technology to prolong in vivo drug actions, decrease drug metabolism, and reduce drug toxicity. Carriers are also used in designs to increase the effectiveness of drug delivery to the target sites of pharmacological actions (U.S. National Library of Medicine. National Institutes of Health). Adjuvant is a substance added to a drug product formulation that affects the action of the active ingredient in a predictable way. Vehicle is an excipient or a substance, preferably without therapeutic action, used as a medium to give bulk for the administration of medicines (Stedman's Medical Spellchecker, © 2006 Lippincott Williams & Wilkins). Such pharmaceutical carriers, adjuvants or vehicles can be sterile liquids, such as water and oils, including those of petroleum, animal, vegetable or synthetic origin, such as peanut oil, soybean oil, mineral oil, sesame oil and the like, excipients, disgregants, wetting agents or diluents. Suitable pharmaceutical carriers are described in "Remington's Pharmaceutical Sciences" by E.W. Martin. The selection of these excipients and the amounts to be used will depend on the form of application of the pharmaceutical composition.

As used herein, the terms "treat", "treating" and "treatment" include in general the eradication, removal, reversion, alleviation, modification, or control of a vulvovaginal dermatologic condition after its onset.

As used herein, the terms "prevention", "preventing", "preventive", "prevent" and "prophylaxis" refer to the capacity of a given substance, composition or medicament to avoid, minimize or difficult the onset or development of a vulvovaginal dermatologic condition before its onset.

Also is disclosed a method of treatment of a patient, notably a woman, suffering a vulvovaginal dermatologic condition, or likely to suffer a vulvovaginal dermatologic condition, which comprises administering to the patient in need of such a treatment or prophylaxis an effective amount of a compound of formulae (I) or (II) or a pharmaceutically acceptable salt, stereoisomer or solvate thereof.

By an "effective" amount or a "therapeutically effective amount" of a drug or pharmacologically active agent is meant a nontoxic but sufficient amount of the drug or agent to provide the desired effect. In the therapy of the present invention, an "effective amount" of ectoine or a derivative thereof is the amount of that compound that is effective to provide the desired effect. The amount that is "effective" will vary from subject to subject, depending on the age and general condition of the individual, the particular active agent or agents, and the like. Thus, it is not always possible to specify an exact "effective amount". However, an appropriate "effective" amount in any individual case may be determined by one of ordinary skill in the art using routine experimentation.

The compound of formulae (I) or (II) can be used together with other additional useful drugs in the prevention and/or treatment of vulvovaginal dermatologic conditions. Said additional drugs can form part of the same pharmaceutical composition or, alternatively, can be provided in the form of a separated composition for their simultaneous or sequential administration to that of the pharmaceutical composition comprising the compound of formulae (I) or (II) or a pharmaceutically acceptable salt or solvate thereof. For example, the treatment with ectoine or an ectoine derivative as proposed in the present invention could be complimented with the administration of corticosteroids (e.g. clobetasol), vaginal moisturizers or lubricants, estrogens, etc.

The following examples are provided as supporting evidence for the invention and should not be taken as a definition of the limits of the same.

### Examples

### 1. Clinical Case: Lichen sclerosus

### A) Presentation

A 59-year-old woman presents to gynecological consultation because of pruritus, vaginal dryness, dysuria and dyspareunia.

Since 2013 the patient repeatedly visited the gynecologist for these same symptoms but she refers increased symptomatology in the last months.

The patient is diagnosed with lichen sclerosus.

### B) Clinical History

Menarche age: 11 years (with regular menstrual cycles until menopause).

Last Menstrual Period: in 2013.

Parity: 2002. Eutocic births at 28 and 34 years.

Contraceptive methods: Hormonal contraceptives for 5 years, and an intrauterine device for 5 years.

No surgical interventions.

Important diseases: Hypercholesterolemia for 8 years controlled with diet.

Toxic habits: very occasional alcohol consumer, and smoker of a pack of cigarettes daily for more than 10 years, ex-smoker for 2 years.

Body Mass Index: 25.15

Pharmacological treatments:
- Clobetasol on demand (current treatment).
- Other treatments previously used related to the pathology include vulvar and vaginal moisturizers, topical methylprednisolone, low dose local estrogens and topical tacrolimus monohydrate.

Other data of interest:
- She refers a balanced diet, she has started with some problems to get to sleep and she does not take any medication at the moment, although she is carrying out blood pressure checks, because lately she has had some increased value.

Psychosexual anamnesis:
- Two stable couples. First couple from 18 to 52 years old, she became a widow and at 54 she started with her current partner.
- The pruritus and dryness alter her daily life and have a negative impact on her sexual relations.
- When she has sex she uses a lubricant but she does not consider it enough.

The gynecological exam carried out provides the following information:
- Vulva inspection. There is a thinning of the labia minora, fissure in the left interlabial fold and dermal changes suggestive of lichen, especially in the posterior labial commissure.

The result of cervico-vaginal cytology is reported as normal.

### C) Therapeutic intervention

In July 2017 the patient was administered local treatment with ectoine, 1/12h morning and night, in the morning vulvar application and at night vulvar and vaginal application. Photos were taken before the start of treatment and after 15 days of treatment (see Figure 1A and 1B, respectively).

### D) Results

After the 15-day treatment with ectoine changes are observed in the coloration of the intralabial folds, the color is more physiological, and the intralabial fissure has disappeared. The patient also reports more moisture, lower pain intensity at the introitus during penetration as well as mild, very localized pruritus (instead of the prior unpleasant sensation of pruritus generalized in the entire vulvar area).

### References

1. GALINSKI, E. A., PFEIFFER, H.-P. and TRÜPER, H. G. (1985), 1,4,5,6-Tetrahydro-2-methyl-4-pyrimidinecarboxylic acid. European Journal of Biochemistry, 149: 135-139

## Claims

1. Compound of formulae (I) or (II) 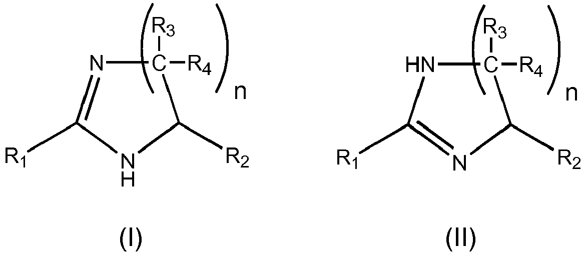
where R¹ is H or C₁-C₄ alkyl,
R² is H, -COOH, -COO(C₁₋C₄ alkyl) or -CONHR⁵, where R⁵ is H, C₁-C₄alkyl, an amino acid radical, dipeptide radical or tripeptide radical,
R³ and R⁴ are in each case independently of one another H or OH,
n is 1, 2 or 3,
or a pharmaceutically acceptable salt, stereoisomer or solvate thereof,
for use in the treatment and/or prevention of a vulvovaginal dermatologic condition selected from the group consisting of vulvovaginal atrophy, lichen sclerosus and Zoon's vulvitis.

2. Compound for use according to claim 1, wherein R¹ is methyl.

3. Compound for use according to any one of the preceding claims, wherein R² is - COOH.

4. Compound for use according to any one of the preceding claims, wherein n is 2.

5. Compound for use according to any one of the preceding claims, wherein the compound is ectoine.

6. Compound for use according to any one of the preceding claims, wherein the compound is hydroxyectoine.

7. Compound for use according to any one of the preceding claims, wherein the vulvovaginal dermatologic condition is lichen sclerosus.

8. A pharmaceutical composition for use in the treatment and/or prevention of a vulvovaginal dermatologic condition selected from the group consisting of vulvovaginal atrophy, lichen sclerosus and Zoon's vulvitis comprising a compound of formula (I) or (II), or a pharmaceutically acceptable salt, stereoisomer or solvate thereof as defined in any one of claims 1 to 6 and a pharmaceutically acceptable excipient.

9. The pharmaceutical composition for use according to claim 8, wherein the vulvovaginal dermatologic condition is lichen sclerosus.

10. The pharmaceutical composition for use according to any one of claims 8 to 9, wherein said composition is for topical administration.

11. The pharmaceutical composition for use according to any one of claims 8 to 10, wherein said composition is selected from the group consisting of a solution, a suspension, a emulsion, a paste, an ointment, a gel, a cream, a lotion, a powder, a soap, a surfactant-containing cleansing preparations, an oil, a foam and a spray.

## Patentansprüche

1. Verbindung der Formeln (I) oder (II) 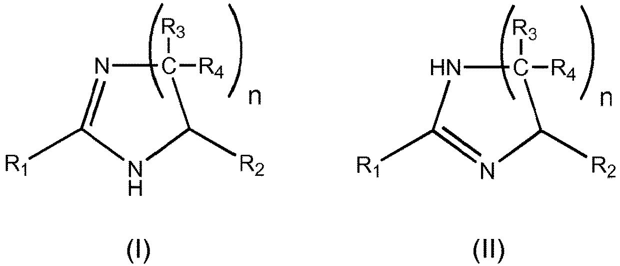
wobei R¹ H oder C₁-C₄Alkyl ist,
R² H, -COOH, -COO(C₁-C₄Alkyl) oder -CONHR⁵ ist, wobei R⁵ H, C₁-C₄ Alkyl, ein Aminosäurerest, Dipeptid-Rest oder Tripeptid-Rest ist, R³ und R⁴ jeweils unabhängig voneinander H oder OH sind,
n 1, 2 oder 3 ist,
oder ein pharmazeutisch akzeptables Salz, Stereoisomer oder Solvat davon,
für die Behandlung und/oder Vorbeugung eines vulvovaginalen dermatologischen Zustandes ausgewählt aus der Gruppe bestehend aus vulvovagianler Atrophie, Lichen sclerosus und Zoon-Vulvitis.

2. Verbindung zur Verwendung nach Anspruch 1 wobei R¹ Methyl ist.

3. Verbindung zur Verwendung nach einem der vorhergehenden Ansprüche, wobei R²-COOH ist.

4. Verbindung zur Verwendung nach einem der vorhergehenden Ansprüche, wobei n 2 ist.

5. Verbindung zur Verwendung nach einem der vorhergehendenden Ansprüche, wobei die Verbindung Ectoin ist.

6. Verbindung zur Verwendung nach einem der vorhergehenden Ansprüche, wobei die Verbindung Hydroxyectoin ist.

7. Verbindung zur Verwendung nach einem der vorhergehenden Ansprüche, wobei der vulvovaginale dermatologische Zustand Lichen sclerosus ist.

8. Pharmazeutische Zusammensetzung zur Behandlung und/oder Vorbeugung eines vulvovaginalen dermatologischen Zustandes, ausgewählt aus der Gruppe bestehend aus vulvovaginaler Atrophie, Lichen sclerosus und Zoon-Vulvitis umfassend eine Verbindung der Formel (I) oder (II), oder ein pharmazeutisch akzeptables Salz, Stereoisomer oder Solvat davon wie in einem der Ansprüche 1 bis 6 definiert ist und einen pharmazeutisch akzeptablen Hilfsstoff.

9. Pharmazeutische Zusammensetzung zur Verwendung nach Anspruch 8, wobei der vulvovaginale dermatologische Zustand Lichen sclerosus ist.

10. Pharmazeutische Zusammensetzung zur Verwendung nach einem der Ansprüche 8 bis 9, wobei die Zusammensetzung zur tropischen Verabreichung ist.

11. Pharmazeutische Zusammensetzung zur Verwendung nach einem der Ansprüche 8 bis 10, wobei die Zusammensetzung ausgewählt ist aus der Gruppe bestehed aus einer Lösung, einer Suspension, einer Emulsion, einer Paste, einer Salbe, einem Gel, einer Creme, einer Lotion, einem Pulver, einer Seife, einem tensidhaltigen Reinigungsmittel, einem Öl, einem Schaum und einem Spray.

## Revendications

1. Composé de formules (I) ou (II) 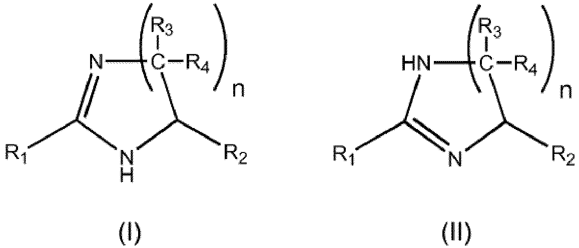
où R¹ est H ou un alkyle en C₁-C₄,
R² est H, -COOH, -COO(alkyle en C₁-C₄) ou -CONHR⁵, où R⁵ est H, un alkyle en C₁-C₄, un radical acide aminé, un radical dipeptide ou un radical tripeptide,
R³ et R⁴ sont dans chaque cas indépendamment l'un de l'autre H ou OH,
n vaut 1, 2 ou 3,
ou un sel, stéréoisomère ou solvate pharmaceutiquement acceptable de celui-ci,
pour utilisation dans le traitement et/ou la prévention d'une pathologie dermatologique vulvovaginale choisie dans le groupe constitué par l'atrophie vulvovaginale, le lichen scléreux et la vulvite de Zoon.

2. Composé pour utilisation selon la revendication 1, où R¹ est un méthyle.

3. Composé pour utilisation selon l'une quelconque des revendications précédentes, où R² est -COOH.

4. Composé pour utilisation selon l'une quelconque des revendications précédentes, où n vaut 2.

5. Composé pour utilisation selon l'une quelconque des revendications précédentes, où le composé est l'ectoïne.

6. Composé pour utilisation selon l'une quelconque des revendications précédentes, où le composé est l'hydroxyectoïne.

7. Composé pour utilisation selon l'une quelconque des revendications précédentes, où la pathologie dermatologique vulvovaginale est le lichen scléreux.

8. Composition pharmaceutique pour utilisation dans le traitement et/ou la prévention d'une pathologie dermatologique vulvovaginale choisie dans le groupe constitué par l'atrophie vulvovaginale, le lichen scléreux et la vulvite de Zoon, comprenant un composé de formule (I) ou (II), ou un sel, stéréoisomère ou solvate pharmaceutiquement acceptable de celui-ci, tel que défini dans l'une quelconque des revendications 1 à 6, et un excipient pharmaceutiquement acceptable.

9. Composition pharmaceutique pour utilisation selon la revendication 8, où la pathologie dermatologique vulvovaginale est le lichen scléreux.

10. Composition pharmaceutique pour utilisation selon l'une quelconque des revendications 8 à 9, où ladite composition est destinée à une administration topique.

11. Composition pharmaceutique pour utilisation selon l'une quelconque des revendications 8 à 10, où ladite composition est choisie dans le groupe constitué par une solution, une suspension, une émulsion, une pâte, un onguent, un gel, une crème, une lotion, une poudre, un savon, des préparations nettoyantes contenant un agent tensioactif, une huile, une mousse et un aérosol.
